(19) Europäisches Patentamt
European Patent Office
Office européen des brevets

(11) Veröffentlichungsnummer: **0 007 609**
**B1**

(12) **EUROPÄISCHE PATENTSCHRIFT**

(45) Veröffentlichungstag der Patentschrift:
**25.11.81**

(51) Int. Cl.³: **C 07 C 47/02,** C 07 C 45/50, C 07 C 45/78

(21) Anmeldenummer: **79102604.0**

(22) Anmeldetag: **24.07.79**

(54) **Verfahren zur Herstellung von Aldehyden.**

(30) Priorität: **31.07.78 DE 2833538**

(43) Veröffentlichungstag der Anmeldung:
**06.02.80 Patentblatt 80/3**

(45) Bekanntmachung des Hinweises auf die Patenterteilung:
**25.11.81 Patentblatt 81/47**

(84) Benannte Vertragsstaaten:
**BE FR GB**

(56) Entgegenhaltungen:
**DE-A-2 055 539**
**DE-B-1 768 391**
**DE-C-933 338**

(73) Patentinhaber: **Ruhrchemie Aktiengesellschaft, Bruchstrasse 219, D-4200 Oberhausen 13 (DE)**

(72) Erfinder: **Bahrmann, Helmut, Dr. Dipl.-Chem., Im Freihof 56, D-4224 Hünxe (DE)**
Erfinder: **Cornils, Boy, Dr. Dipl.-Chem., Friedrich-Ebert-Strasse 45, D-4220 Dinslaken (DE)**
Erfinder: **Diekhaus, Gerhard, Dr. Dipl.-Chem., Walsumermarkstrasse 89, D-4200 Oberhausen 14 (DE)**
Erfinder: **Kascha, Waldemar, Franzstrasse 22, D-4200 Oberhausen 14 (DE)**
Erfinder: **Weber, Jürgen, Dr. Dipl.-Chem., Bunsenstrasse 17, D-4200 Oberhausen 13 (DE)**

**EP 0 007 609 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Aldehyden

Die vorliegende Erfindung betrifft ein Verfahren zur Herstellung von Aldehyden der allgemeinen Formel

$$R-CH(CH_3)CHO$$

durch Umsetzung von Olefinen der allgemeinen Formel

$$R-CH=CH_2$$

mit Kohlenmonoxid und Wasserstoff bei erhöhter Temperatur und erhöhtem Druck in Gegenwart von Phosphin enthaltenden Rhodiumkomplexkatalysatoren.

Zur Herstellung von Aldehyden und Alkoholen wird in der Technik in großem Umfang die sogenannte Oxosynthese (Hydroformylierung) angewendet. Als Katalysator für die Umsetzung des Olefins mit Kohlenmonoxid und Wasserstoff dient in technischem Maßstab vor allem Kobalt, das in Form seiner Carbonylverbindungen wirksam ist.

In jüngster Zeit werden insbesondere zur Hydroformylierung spezieller Olefine Rhodiumkomplexkatalysatoren, in denen biphile Liganden wie tertiäre Phosphine mit dem Rhodiumatom, koordiniert sind, zur Hydroformylierung eingesetzt. Rhodiumkatalysatoren zeichnen sich durch hohe Selektivität bezüglich der Bildung geradkettiger Aldehyde aus und erlauben, die Umsetzung unter geringerem Druck als bei herkömmlichen Verfahren durchzuführen.

Nach der DE-OS 1 793 069 wird zur Herstellung von sauerstoffhaltigen, an normalen Aldehyden reichen Produkten eine $\alpha$-olefinische Verbindung mit Kohlenmonoxid und Wasserstoff in Anwesenheit einer katalytischen Menge eines Komplexkatalysators, der im wesentlichen aus Rhodium in komplexer Kombination mit Kohlenmonoxid und einem Liganden besteht, der ein mit drei organischen Reste substituiertes, dreiwertiges Atom eines Elementes aus der Gruppe V enthält, umgesetzt. Der Ligand ist unter anderem dadurch charakterisiert, daß er ein freies Elektronenpaar besitzt und einen $\Delta$-HNP-Wert von mindestens 425 vorzugsweise mindestens 500 hat. Der $\Delta$-HNP-Wert ist ein Maß der Basizität des Liganden, hohe Basizität entspricht dabei einem niedrigen $\Delta$-HNP-Wert. Die Umsetzung wird bei 50 bis 145°C und einem Gesamtdruck von Kohlenmonoxid und Wasserstoff von weniger als etwa 30,9 bar durchgeführt.

Den Vorteilen der durch Rhodiumkomplexverbindungen katalysiertes Oxoreaktion, insbesondere Anwendung niedrigen Drucks und hohe Ausbeuten an geradkettigen Aldehyden steht als Nachteil u. a. das Erfordernis, große Reaktionsräume einsetzen zu müssen, entgegen wie auch die Schwierigkeit, in $\alpha$-Stellung methylverzweigte Aldehyde in zufriedenstellenden Ausbeuten und hoher Reinheit zu gewinnen.

Es bestand daher die Aufgabe, ein Verfahren zu entwickeln, mit dessen Hilfe es gelingt, in $\alpha$-Stellung verzweigte höhere Aldehyde, d. h. Aldehyde mit mindestens 10 Kohlenstoffatomen in einfacher Weise darzustellen.

Es wurde gefunden, daß man Aldehyde der allgemeinen Formel

$$R-CH(CH_3)-CHO,$$

wobei R für geradkettige Alkylreste mit 7 bis 12 Kohlenstoffatomen steht, dadurch erhält, daß man Olefine der allgemeinen Formel

$$R-CH=CH_2,$$

in der R der vorstehenden Definition entspricht, mit Kohlenmonoxid und Wasserstoff bei 80 bis 170°C und 200 bis 350 bar in Gegenwart von Phosphin enthaltenden komplexen Rhodiumverbindungen als Katalysatoren umsetzt, die als solche dem Reaktionsgemisch zugegeben oder unter den Reaktionsbedingungen aus im Olefin löslichen Rhodiumsalzen, organischen Phosphinen, Kohlenmonoxid und Wasserstoff gebildet werden und danach das Reaktionsprodukt bei 100 bis 190°C gegebenenfalls nach vorheriger Abtrennung des Katalysators thermisch behandelt.

Das neue Verfahren weist einen einfachen Weg, aus dem normale (geradkettige) und in $\alpha$-Stellung methylverzweigte Aldehyde enthaltenden Reaktionsgemisch die $\alpha$-verzweigten Aldehyde zu gewinnen. Die Hydroformylierung unter den erfindungsgemäßen Bedingungen stellt sicher, daß das Ausgangsolefin in hoher Ausbeute, mit großer Reaktionsgeschwindigkeit und in hoher Raumzeitausbeute zu einem Gemisch von unverzweigten Aldehyden und solchen, die in $\alpha$-Stellung eine Methylgruppe enthalten, umgesetzt wird. Durch die thermische Nachbehandlung werden auf einfachem Wege die beiden isomeren Aldehyde getrennt. Überraschenderweise wandeln sich nämlich die geradkettigen Aldehyde der genannten Kohlenstoffatomzahl bei Erhitzen auf 100 bis 190°C in höhermolekulare Kondensationsprodukte um, während die $\alpha$-methylverzweigten Aldehyde unverändert erhalten bleiben.

Zur Umsetzung der Olefine wendet man Mischungen an, die Kohlenmonoxid und Wasserstoff in der Regel im Volumverhältnis von 1 : 4 bis 4 : 1, insbesondere 1 : 2 bis 2 : 1 enthalten.

Die Reaktion wird bei Temperaturen von 80 bis 170°C und Drücken von 200 bis 350 bar durchgeführt. Besonders bewährt haben sich Temperaturen von 100 bis 150°C und Drücke von 200 bis 270 bar.

Als Katalysatoren finden komplexe Rhodiumverbindungen Anwendung, die neben Kohlenmonoxid und Wasserstoff organische Phosphine als Liganden enthalten. Diese komplexen Verbindungen werden entweder vorgebildet dem Reaktionsgemisch zugesetzt oder aber unter den Reaktionsbedingungen aus im Olefin löslichen Rhodiumsalzen, organischen Phosphinen, Kohlenmonoxid und Wasserstoff gebildet. Üblicherweise liegt der Katalysator in einer Komzentration von 20 bis 100 ppm Rhodium, berechnet als Metall, bezogen auf das eingesetzte Olefin vor. Als Rhodium-Verbindungen, die in situ in den Rhodiumkomplex umgewandelt werden, kommen insbesondere Rhodiumsalze von Fettsäuren, d. h. Carbonsäuren mit mindestens 6 bis etwa 20 Kohlenstoffatomen in Betracht. Beispiele für derartige Salze sind Rh-2-ethylhexanoat, Rh-stearat und Rh-oleat.

Mit besonderem Erfolg werden als Komplexliganden organische Phosphine verwendet, deren Elektronen Donator-Acceptor-Verhalten durch einen $\Sigma\chi_i$-Wert zwischen 0 und 14 charakterisiert ist. Die Bedeutung des $\Sigma\chi_i$-Wertes für homogen katalysierte Reaktionen ist in der Arbeit von Chadwick A. Tolman in J. Am. Soc. 92 (1970), S. 2953—2956 näher beschrieben. Zu den Phosphinen mit den genannten Eigenschaften gehören insbesondere Trialkylphosphine z. B. Triethylphosphin, Tributylphosphin, Trioctylphosphin sowie Triphenylphosphin.

Rhodiumkomplexe, die neben Kohlenmonoxid und gegebenenfalls Wasserstoff auch Phosphin enthalten, sind stabiler als solche Komplexe, die lediglich aus Rhodium, Kohlenmonoxid und gegebenenfalls Wasserstoff aufgebaut sind. Um einen Katalysator mit ausreichender Stabilität zu erhalten, setzt man daher vorzugsweise Phosphin im Überschuß ein, so daß je g-Atom Rhodium mindestens 50 Mol Phosphin im Reaktionsgemisch vorhanden sind. Besonders bewährt hat es sich, je g Atom Rhodium 50 bis 200 Mole Phosphin anzuwenden.

Die Umsetzung kann ohne Mitverwendung zusätzlicher Lösungsmittel durchgeführt werden. In diesem Fall dienen die Ausgangsolefine und das Phosphin als Reaktionsmedium. Man kann aber die Umsetzung auch in einem Lösungsmittel vornehmen, wobei man z. B. aromatische Kohlenwasserstoffe, wie Benzol, Toluol, Xylol oder höhere aliphatische Kohlenwasserstoffe wie Pentan, Hexan, ferner Äther oder Alkohole verwendet.

Bei der Umsetzung von Olefinen der allgemeinen Formel

$$R-\overset{\beta}{C}H=\overset{\alpha}{C}H_2$$

mit Kohlenmonoxid und Wasserstoff findet die Hydroformylierung bevorzugt in $\alpha$-Stellung statt, man erhält also vor allem geradkettige Aldehyde. Beim Eintritt der Formylgruppe in $\beta$-Stellung werden methylverzweigte Aldehyde gebildet. Normalerweise besteht das Reaktionsgemisch unter den genannten Reaktionsbedingungen zu etwa 60 Gew.-% aus geradkettigem Aldehyd und zu etwa 40 Gew.-% aus verzweigten Aldehyden, wobei neben Aldehyden, die in $\alpha$-Stellung eine Methylgruppe aufweisen, durch Wanderung der Doppelbindung in größeren Anteilen auch weitere Isomere entstehen, die durch Hydroformylierung der durch Doppelbindungsisomerisierung gebildeten innenständigen Olefine entstehen.

Es hat sich überraschenderweise gezeigt, daß bei Einsatz von Rhodiumkatalysatoren, die als Komplexliganden organische Phosphine mit einem $\Sigma\chi_i$-Wert zwischen 0 und 5 enthalten, die Wanderung der Doppelbindung im Einsatzolefin völlig unterdrückt wird, so daß neben dem geradkettigen Aldehyd ausschließlich der Aldehyd gleicher Kohlenstoffatomzahl mit einer Methylgruppe in $\alpha$-Stellung zur Carbonylgruppe gebildet wird. Die Trennung beider isomerer Aldehyde gelingt durch eine im Anschluß an die eigentliche Synthese durchgeführte thermische Behandlung des Reaktionsproduktes. Ihr kann eine Abtrennung des Katalysators nach bekannten Verfahren, z. B. durch Umsetzung des Reaktionsgemisches mit Wasserstoff oder Wasserdampf vorausgehen.

Zur thermischen Behandlung wird das Reaktionsgemisch einfach auf Temperaturen zwischen 100 bis 190°C erhitzt. Dieses Erhitzen kann gesondert durchgeführt werden, zweckmäßiger wird es jedoch mit der Destillation des Reaktionsgemisches verknüpft. In diesem Falle leitet man das aus der Synthese kommende Gemisch einer Destillationskolonne zu und erhitzt auf die entsprechenden Temperaturen, wobei die verzweigten Aldehyde abdestilliert werden, während die geradkettigen Aldehyde durch Kondensation in schwerflüchtige Verbindungen übergehen und im Sumpf der Kolonne zurückbleiben.

Dem erfindungsgemäßen Prozeß führt man z. B. in der Weise durch, daß man das Olefin zusammen mit einem Rhodiumsalz und einem Phosphin und gegebenenfalls mit einem Lösungsmittel in einen Reaktor vorlegt und mit Kohlenmonoxid und Wasserstoff unter den beschriebenen Temperatur- und Druckbedingungen umsetzt. In geeigneten Apparaten läßt sich die Reaktion auch kontinuierlich ausführen. Nach dem Erkalten und Entspannen des Reaktionsproduktes wird das überflüssige Gasgemisch abgetrennt und der Reaktionsaustrag der thermischen Behandlung unterworfen.

Die nach dem erfindungsgemäßen Verfahren herstellbaren $\alpha$-Methyl-alkylaldehyde finden als Bestandteile von Parfümkompositionen Verwendung.

In den folgenden Beispielen wird das erfindungsgemäße Verfahren veranschaulicht.

## Beispiel 1

### Hydroformylierung von n-Undecen 1

In einem Edelstahl-Autoklaven werden 750 g (4,77 Mol) n-Decen-1 in Gegenwart von 18,4 mg (0,18 mMol) Rhodium in Form des Rhodium-2-äthylhexanoats und 3,5 g (17,3 mMol) Tri-n-butylphosphin bei 130°C und 270 bar mit Kohlenmon-

oxid und Wasserstoff ($CO : H_2 = 1 : 1$) umgesetzt. Nach etwa 1,5 Stunden, wenn keine Gasaufnahme mehr erfolgt, wird der Autoklav mit dem Reaktionsprodukt auf Raumtemperatur abgekühlt. Die daran anschließende destillative Aufarbeitung in einer 36 Bödenkolonne bei 13,33 mbar, liefert bei einer Kopftemperatur von 120°C und einer Sumpftemperatur von 170°C 314 g 2-Methylundecanal entsprechend einer Ausbeute von 35%, bezogen auf eingesetztes n-Undecen-1.

Die gaschromatografisch ermittelte Reinheit des 2-Methylundecanals liegt höher als 98%.

### Beispiel 2

Hydroformylierung von n-Decen-1

In einem Edelstahl-Autoklaven werden 736 g (5,25 Mol) n-Decen-1 mit den gleichen Katalysatormengen und unter den gleichen Reaktionsbedingungen wie in Beispiel 1 hydroformyliert. Nach 2 Stunden ist die Reaktion beendet. Der Autoklav wird abgekühlt und das Reaktionsgemisch in einer Kolonne mit 36 Böden bei 13,33 mbar destilliert. Bei 110°C Kopftemperatur und 145°C gehen 268 g 2-Methyldecanal entsprechend einer Ausbeute von 30%, bezogen auf Decen, über. Die gaschromatografisch ermittelte Reinheit liegt über 98%.

## Patentansprüche

1. Verfahren zur Herstellung von Aldehyden der allgemeinen Formel

$R-CH(CH_3)-CHO$,

wobei R für geradkettige Alkylreste mit 7 bis 12 Kohlenstoffatomen steht, dadurch gekennzeichnet, daß man Olefone der allgemeinen Formel

$R-CH=CH_2$,

in der R der vorstehenden Definition entspricht, mit Kohlenmonoxid und Wasserstoff bei 80 bis 170°C und 200 bis 350 bar in Gegenwart von Phosphin enthaltenden komplexen Rhodiumverbindungen als Katalysatoren, die als solche dem Reaktionsgemisch zugegeben oder unter den Reaktionsbedingungen aus im Olefon löslichen Rhodiumsalzen, organischen Phosphinen, Kohlenmonoxid und Wasserstoff gebildet werden, umsetzt und danach das Reaktionsprodukt bei 100 bis 190°C, gegebenenfalls nach vorheriger Abtrennung des Katalysators, thermisch behandelt.

2. Verfahren nach Anspruch 1, dadurch gekennzeichnet, daß als lösliche Rhodiumsalze Salze von organischen Carbonsäuren mit mindestens 6 Kohlenstoffatomen eingesetzt werden.

3. Verfahren nach Anspruch 1 und 2, dadurch gekennzeichnet, daß der $\Sigma\chi_i$-Wert der organischen Phosphine 0 bis 14, vorzugsweise 0 bis 5 beträgt.

4. Verfahren nach Anspruch 1 bis 3, dadurch gekennzeichnet, daß je g-Atom Rhodium mindestens 50 Mole, vorzugsweise 50 bis 200 Mole organisches Phosphin eingesetzt werden.

## Claims

1. A process for the preparation of aldehydes of the general formula

$R-CH(CH_3)-CHO$

wherein R is a straight-chain alkyl group having 7 to 12 carbon atoms, characterized in that olefins of the general formula

$R-CH=CH_2$

wherein R is as defined above are reacted with carbon monoxide and hydrogen at 80 to 170°C and 200 to 350 bar in the presence of organic phosphine-containing complex rhodium compounds as catalysts, which are added as such to the reaction mixture or are formed under the reaction conditions from rhodium salts which are soluble in the olefin, organic phosphines, carbon monoxide and hydrogen, and the reaction product is subjected thereafter to a thermal treatment at 100 to 190°C, if necessary after previous separation of the catalyst.

2. A process according to claim 1, characterized in that salts of organic carboxylic acids having at least 6 carbon atoms are used as soluble rhodium salts.

3. A process according to claims 1 and 2, characterized in that the organic phosphines have a $\Sigma\chi_i$-value of 0 to 14 and preferably 0 to 5.

4. A process according to claims 1 to 3, characterized in that at least 50 moles and preferably 50 to 200 moles of organic phosphine are used per 1 gram atom of rhodium.

## Revendications

1. Procédé de préparation d'aldéhydes de formule générale

$R-CH(CH_3)-CHO$

dans laquelle R représente des restes alkyle à chaîne droite en $C_7-C_{12}$, caractérisé en ce que l'on fait réagir des oléfines de formule générale

$R-CH=CH_2$

dans laquelle R répond à la définition donnée ci-dessus, avec l'oxyde de carbone et l'hydrogène à une température de 80 à 170°C et une pression de 200 à 350 bars en présence de composés complexes du rhodium contenant une phosphine qui servent de catalyseurs qu'on

introduit tels quels dans le mélange de réaction ou qu'on forme dans les conditions de la réaction à partir de sels de rhodium solubles dans l'oléfine, de phosphines organiques, d'oxyde de carbone et d'hydrogène, puis on soumet le produit de réaction à un traitement thermique à une température de 100 à 190° C, le cas échéant, après séparation du catalyseur.

2. Procédé selon la revendication 1, caractérisé en ce que l'on utilise en tant que sels solubles de rhodium, des sels d'acides organiques carboxyliques à au moins 6 atomes de carbone.

3. Procédé selon les revendications 1 et 2, caractérisé en ce que la valeur de $\Sigma\chi_i$ des phosphines organiques va de 0 à 14, de préférence de 0 à 5.

4. Procédé selon les revendication 1 à 3, caractérisé en ce que, pour un atome-gramme de rhodium, on utilise au moins 50 moles, de préférence 50 à 200 moles de phosphine organique.